# EUROPEAN PATENT SPECIFICATION

(11) **EP 1 928 512 B1**
(45) Date of publication and mention of the grant of the patent: **14.11.2012**
(21) Application number: 06814079.7
(22) Date of filing: 01.09.2006
(51) Int. Cl.: A61L 27/36, A61L 27/50

(54) **ATTACHMENT OF MATERIAL TO AN IMPLANTABLE FRAME BY CROSS-LINKING**
BEFESTIGUNG VON MATERIAL AN EINEM IMPLANTIERBAREN RAHMEN DURCH VERNETZUNG
FIXATION DE MATERIAU A UN CADRE IMPLANTABLE PAR RETICULATION

(30) Priority: 01.09.2005 US 713453 P
(43) Date of publication of application: 11.06.2008
(73) Proprietor: Cook Medical Technologies LLC, Bloomington, IN 47404 (US)
(72) Inventor: RAM, Paul, H., Bloomington, IN 47403 (US); CASE, Brian, C., Lake Villa, IL 6046 (US); HOFFMAN, Grant, T., Bloomington, IN 47401 (US)
(74) Representative: Ellis, Katherine Elizabeth Sleigh
(86) International application number: PCT/US2006/034276
(87) International publication number: WO 2007/028052

(56) References cited:
- US-A1- 2001 039 450
- US-A1- 2003 181 968
- US-A1- 2003 181 974
- US-A1- 2004 024 452
- US-B1- 6 187 039

## Description

The present invention relates to medical devices. More particularly, the invention relates to medical devices for implantation in a body vessel comprising a material attached to a frame.

Many vessels in animals transport fluids from one body location to another. Frequently, fluid flows in a substantially unidirectional manner along the length of the vessel. For example, veins in the body transport blood to the heart and arteries carry blood away from the heart.

Recently, various implantable medical devices and minimally invasive methods for implantation of these devices have been developed to deliver these medical devices within the lumen of a body vessel. These devices are advantageously inserted intravascularly, for example from an implantation catheter. For example, implantable medical devices can function as a replacement venous valve, or restore native venous valve function by bringing incompetent valve leaflets into closer proximity. Such devices can comprise an expandable frame configured for implantation in the lumen of a body vessel, such as a vein. Venous valve devices can further comprise features that provide a valve function, such as opposable leaflets.

Many devices, such as implantable medical devices, comprise a material attached to a frame. The inclusion of a material to a medical device often requires a connection between the frame and material. The dynamic environment in which the medical device is placed requires a connection that maintains the material in a desired structured orientation with respect to the frame.

There exists a need in the art for an implantable medical device that comprises a material suitably attached to a frame. Materials attached to an implantable frame without use of adhesives are particularly desirable. For example, a portion of material can be joined to an implantable frame by treating the material and/or frame with conditions that promote the formation of cross-link chemical bonds that suitably secure portions of the material to a frame.

The invention relates to medical devices for implantation in a body vessel.

According to a first aspect of the present invention, there is provided a medical device as specified in claim 1.

The cross-linked region can comprise cross-linkable material attached to itself. The cross-linked region can allow cross-linkable material to move relative to the frame to any suitable extent or any suitable direction. Cross-linkable material can include graft material or valve leaflet. Preferably, the cross-linkable material is a flexible material.

The invention includes embodiments within the scope of the claims and is limited only by the claims made by the Applicants. Additional understanding of the invention can be obtained by referencing the detailed description of embodiments of the invention, below, and the appended drawings.

**Figure 1A and 1B** is a partially sectioned side view of a medical device according to an embodiment of the present invention;

**Figure 2** is an enlarged cross-sectional view of the cross-linked region taken along line A-A'.

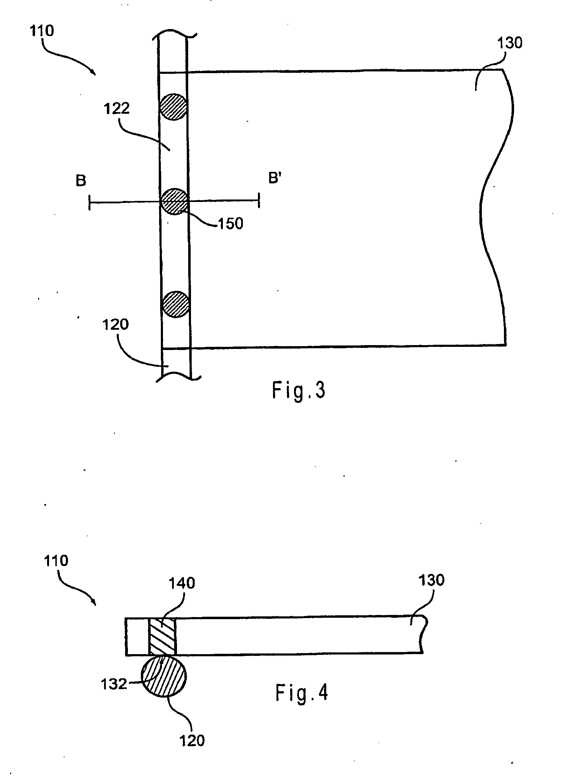

**Figure 3** is a partially sectioned side view of a medical device according to an embodiment of the present invention.

**Figure 4** is an enlarged cross-sectional view of the cross-linked region taken along line B-B'.

**Figure 5** is a side view of a valve according to an embodiment of the present invention having cross-linkable material attached to a frame.

**Figure 6** is a left side view of the valve depicted in **Figure 5**.

**Figure 7** is a side view of a valve according to an embodiment of the present invention.

**Figure 8** is an enlarged cross-sectional view of the cross-linked region taken along line C-C'.

**Figure 9** is an enlarged cross-sectional view of the cross-linked region taken along line C-C'.

**Figure 10** is an enlarged sectional view of the cross-linked region taken along line D-D'.

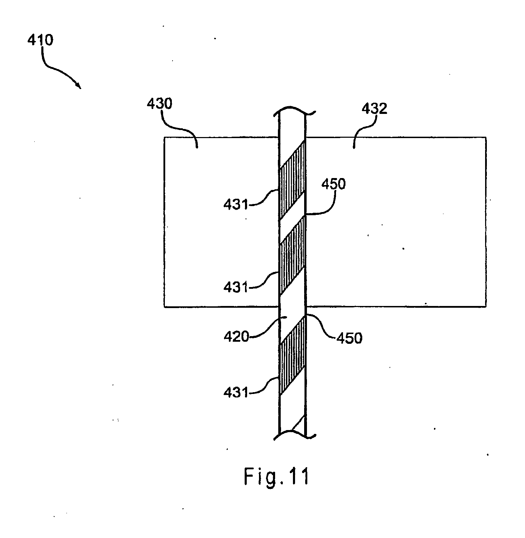

**Figure 11** is a partially sectioned side view of a medical device according to an embodiment of the present invention.

**Figure 12** is a perspective view of a medical device according to an embodiment of the present invention.

**Figure 13** is an enlarged cross-sectional view of the cross-linked region taken along line E-E'.

**Figure 14** is a perspective view of a medical device according to an embodiment of the present invention.

**Figure 15** is a perspective view of a medical device according to an embodiment of the present invention in a closed configuration.

**Figure 16** is a perspective view of a medical device according to an embodiment of the present invention in an open configuration.

### DETAILED DESCRIPTION OF EMBODIMENTS

The following detailed description and appended drawings describe and illustrate various exemplary embodiments of the invention. The description and drawings serve to enable one skilled in the art to make and use the invention, and are not intended to limit the scope of the invention in any manner.

The invention provides medical devices for implantation in a body vessel, methods of making the medical devices, and methods of treatment that utilize the medical devices.

As used herein the terms "comprise(s)," "include(s)," "having," "has," "contain(s)," and variants thereof, are intended to be open-ended transitional phrases, terms, or words that do not preclude the possibility of additional acts or structure.

As used herein, the term "body vessel" means any tube-shaped body passage lumen that conducts fluid, including but not limited to blood vessels such as those of the human vasculature system, biliary ducts and ureteral passages.

As used herein, the term "implantable" refers to an ability of a medical device to be positioned at a location within a body, such as within a body vessel. Furthermore, the terms "implantation" and "implanted" refer to the positioning of a medical device at a location within a body, such as within a body vessel.

As used herein, "endolumenally," "intraluminally" or "transluminal" all refer synonymously to implantation placement by procedures wherein the prosthesis is advanced within and through the lumen of a body vessel from a remote location to a target site within the body vessel. In vascular procedures, a medical device will typically be introduced "endovascularly" using a catheter over a guidewire under fluoroscopic guidance. The catheters and guidewires may be introduced through conventional access sites to the vascular system, such as through the femoral artery, or brachial and subclavian arteries, for access to the coronary arteries.

A "biocompatible" material is a material that is compatible with living tissue or a living system by not being toxic or injurious and not causing immunological rejection.

The invention relates to medical devices for implantation in a body vessel. More specifically, embodiments of the invention relate to a medical device comprising a cross-linkable material maintained in connection to a frame by one or more points of attachment.

The term "cross-link" refers to the formation of a chemical bond between two polymers or molecules by formation of one or more covalent bonds between the two polymers or molecules. Preferably, a medical device comprises a flexible material comprising a polymer capable of forming cross-link bonds to itself or to another polymer or molecule.

The term "frame" refers to any biocompatible frame suitable for implantation within a body vessel. Preferably, a frame can expand from a compressed, or unexpanded, delivery configuration to one or more radially expanded deployment configurations, for example through self-expansion or balloon expansion of the frame. The expanded configuration can have any suitable cross-sectional configuration, including circular or elliptical. In one embodiment, the frame can be oriented along the longitudinal axis of a body vessel in the expanded or compressed configurations.

As used herein, a "cross-linkable material" refers to any material suitable for attachment about a frame of a medical device. Preferably, the cross-linkable material maintains flexibility to move in response to fluid contacting the material in a body vessel.

As used herein, a "point of attachment" refers to a location wherein a bond is formed thereby linking two adjacent surfaces.

As used herein, a "cross-linked region" refers to a region comprising a first region and second region that are bonded together via cross-linking.

### CROSS-LINKED REGIONS AND POINTS OF ATTACHMENT

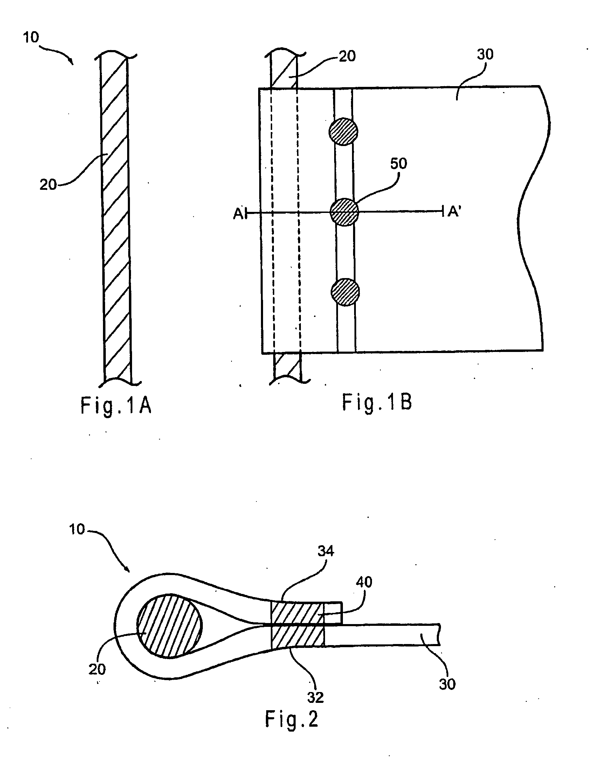

Referring to **Figure 1A** and **Figure 1B**, a medical device **10** is provided for implantation within a vessel of a body lumen. The medical device **10** of **Fig. 1B** illustrates a partially sectioned side view of a medical device according to an embodiment of the present invention. **Figure 2** illustrates an enlarged cross-sectional view of the cross-linked region taken along line A-A'of **Fig. 1B** which includes the cross-linked region **40** forming a portion of point of attachment **50**, and as well as a portion of cross-linkable material **30** and a portion of frame **20**. The medical device **10** includes a frame **20** and a cross-linkable material **30** having at least one cross-linked region **40** and a non cross-linked region, where the cross-linked region **40** maintains the cross-linkable material **30** in connection to the frame **20** (see **Fig. 2**). Cross-linkable material **30** is attached to frame **20** by one or more points of attachment **50**. Points of attachment **50** are formed by one or more cross-linked regions **40** of the cross-linkable material **30**. The cross-linkable material **30** is cross-linked to itself to form a point of attachment **50**. The point of attachment **50** is moveable relative to the frame **20**.

**Figure 1** illustrates a suitable frame **20** for use in a medical device **10** according to an embodiment of the present invention. Referring to **Fig. 2****,** the cross-linkable material **30** is attached to itself to maintain the connection to the frame **20.** The cross-linkable material **30** preferably is suitable for use as a prosthetic valve leaflet. The cross-linkable material **30** is attached to itself by cross-linking a first region **32** of the cross-linkable material **30** to a second region **34** of the cross-linkable material **30.** A point of attachment **50** is formed along a portion of the cross-linkable material **30** wherein the first region **32** of the cross-linkable material **30** is bonded to the second region **34** of the cross-linkable material **30**.

**Figure 2** illustrates a cross linked region **40** including cross-linkable bonds between the first region **32** of the cross-linkable material **30** and the second region **34** of the cross-linkable material **30**. The frame **20** is positioned between the first region **32** of the cross-linkable material **30** and second region **34** of the cross-linkable material **30**, wherein the cross-linkable material **30** remains movable about the frame **20**. The cross-linkable material **30** is overlapped over the frame **20** which allows the cross-linkable material **30** to remain movable with respect to the frame **20**.

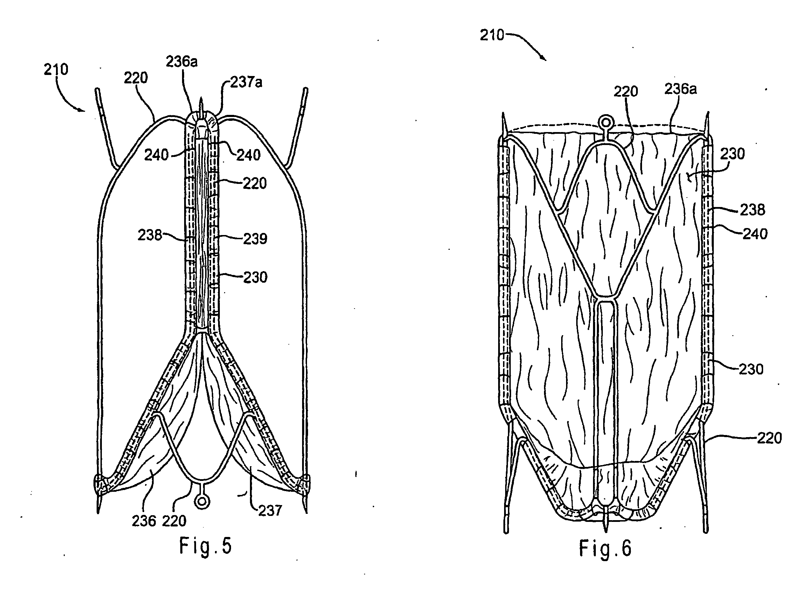

With reference to **Figures 5-6**, various side views of a valve **210** are illustrated according to an embodiment of the invention having cross-linkable material **230** secured to a frame **220**. The medical valve **210** further provides two leaflets **236** and **237** in a first configuration for implantation in a patient. In particular, **Figure 5** provides a side view of a valve **210** viewed in a direction parallel to the coapting upper edges **236a** and **237a** of leaflets **236** and **237**. **Figure 6** provides a view of the valve **210** depicted in **Figure** 5 taken from one end.

As can be seen from **Figures 5-6**, leaflets **236** and **237** include respective free edges **236a** and **237a** for coaptation with one another and respective fixed edges **238** and **239** the central portion of which can be displaced toward the wall of a vascular vessel upon implantation of valve **210** in a body vessel with fluid flowing therethrough.

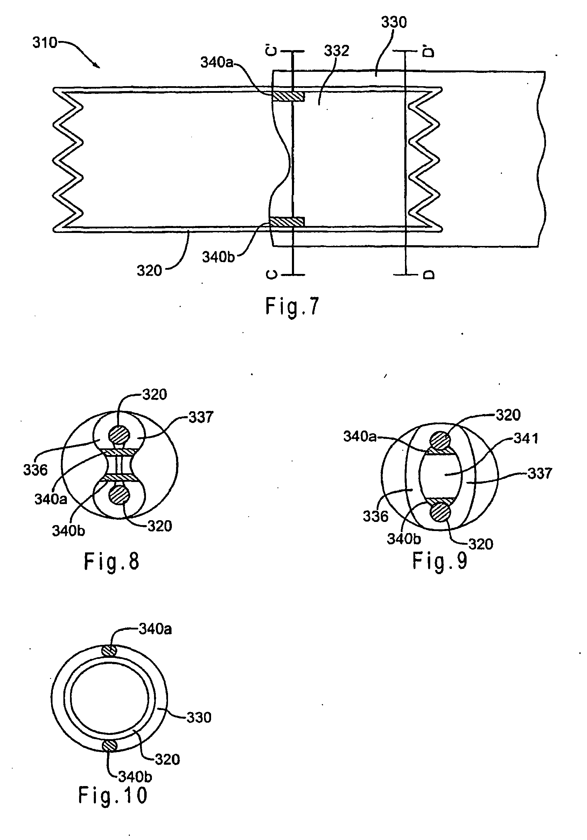

**Figure 7** illustrates an alternate valve **310** according to another embodiment of the invention. In this embodiment, a cross-linkable material **330** is attached to a frame **320** to maintain a connection to the frame **320.** The cross-linkable material **330** extends along approximately half of frame **320.**

**Figures 8-9** illustrate a cross-sectional view of the valve shown in **Fig. 7** taken along line C-C, wherein the cross-linkable material **330** defines leaflets **336, 337.** **Figure 10** illustrates a sectional view of the cross-linked region taken along line D-D'. The leaflets are movable between a first and second position when the valve **310** is placed in a body lumen. In the first position, illustrated in **Figure 8****,** the leaflets **336, 337** permit fluid flow in a first direction. The pressure created by the flow of fluid exerts a force on one face of the leaflets **336, 337,** displacing the leaflets **336, 337** toward a closed position. In the second position, illustrated in **Figure 9****,** the leaflets **336, 337** substantially prevent fluid flow in a second, opposite direction. When a pressure change and/or flow reversal occurs, the pressure created by the flow of fluid exerts a force on an opposite face of the leaflets **336, 337** displacing the leaflets **336, 337** toward an open position. By moving between these two positions, the leaflets **336, 337** provide a valving function to the valve **310,** allowing it to regulate fluid flow. The leaflets **336, 337** further define a valve opening **341.** The leaflets **336, 337** are movable between the first and second positions. While in the first position the leaflets **336, 337** come together to close the opening **341.** In the second position, the opening **341** allows blood flow through the valve.

The dimensions and configurations of the cross-linkable material **330** can be optimized based upon the vessel in which the valve **310** is placed. The size and configuration selected will depend on several factors, including the vessel size, typical flow volume and rates, and other factors depending on the configuration of the valve **310**.

The cross-linkable material can include a combination of tissue and polymers depending on the particular design and configuration. For example, the cross-linkable material located on the frame can include an extra cellular matrix material, such as small intestine submucosa (SIS), while the remaining cross-linkable material can comprise a tissue. The frame can comprise one or more suitable polymers using conventional manufacturing techniques, such as braiding, coiling, or knitting.

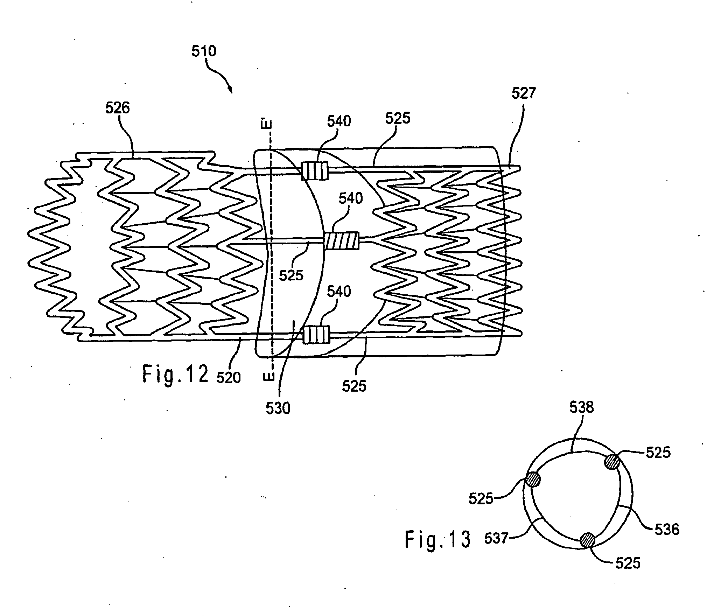

**Figure 12** illustrates a medical device **510** according to another embodiment of the present invention. The medical device **510** comprises a frame **520** having a cross-linkable material **530** cross-linked to the frame **520**. The structure of the frame **520** consists of three bar members **525**. In the embodiment, the cross-linkable material **530** is cross-linked to the bar members **525** of the frame **520**. The frame **520** further includes a proximal portion **526** and a distal portion **527** connected to the bar members **525** of the frame **520**. The proximal portion **526** and distal portion **527** of the frame **520** can include a Z or S shaped configuration, however, the configuration should not be construed as limiting the scope of invention. One of ordinary skill in the art would understand that other geometries may be used.

The cross-linkable material **530** can be attached to itself at a location between the proximal portion **526** and distal portion **527** of the medical device **510**. The cross-linkable material **530** is preferably located at the approximate longitudinal midpoint of the bar members **525** and extend to an end of the device **510** (see **FIG. 12**). In an alternate embodiment, the cross-linkable material **530** can cover the entire length of the medical device **510.** The cross-linkable material **530** can further define a plurality of leaflets **536, 537, 538** wherein each leaflet **536, 537**, **538** is cross-linked to a separate bar member **525**. **Figures 13** illustrates a cross-sectional view of the device **510** shown in **Fig. 12**, wherein the number of leaflets **536**, **537**, **538** is equivalent to the number of bar members **525**. In the illustration, the device **510** includes three leaflets and three bar members, however, in an alternate embodiment the number of leaflets and/or bar members may be larger or smaller.

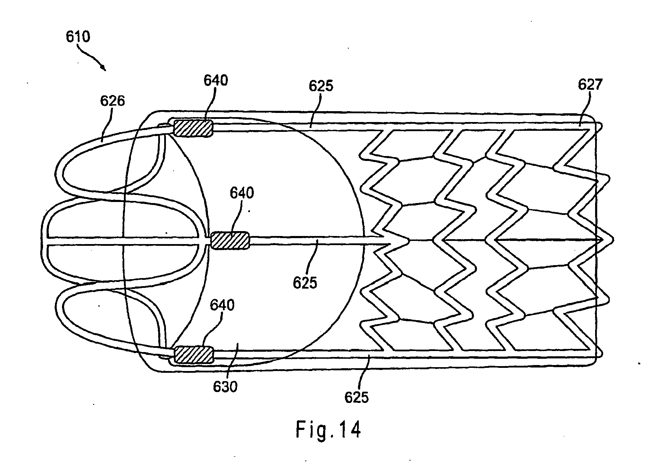

**Figure 14** illustrates a medical device **610** according to another embodiment of the present invention. In this embodiment, a frame **620** includes a proximal portion **626** having an open end and a distal portion **627** connected to bar members **625** of the frame **620**. The proximal portion **626** has an S shaped configuration and the distal portion **627** has a Z shaped configuration, however, the configuration should not be construed as limiting the scope of invention.

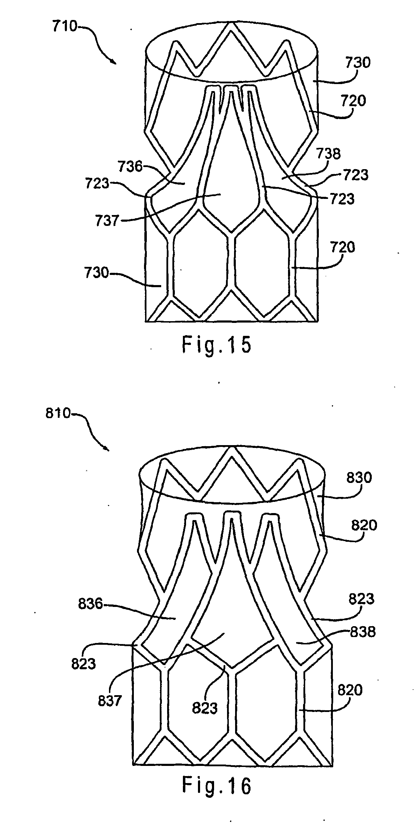

**Figures 15-16** illustrate a medical device **710** according to another embodiment of the present invention. In this embodiment, the cross-linkable material **730** defines a plurality of leaflets **736, 737, 738** wherein the leaflets are movable between a first and second position when the device **710** is placed in a body lumen. In the first position (see **FIG. 14**), the leaflets **736, 737, 738** come together to close a valve opening defined by the leaflets **736, 737, 738.** In the second position (see **FIG. 15**), the leaflets **736, 737, 738** open the valve opening which allows blood flow through the valve. By moving between these two positions, the leaflets **736, 737, 738** provide a valving function to the medical device **710**, allowing it to regulate fluid flow. The structure of the frame **720** defines a leaf frame **723** corresponding to the shape of the leaflets **736, 737, 738**.

### CROSSLINKING

Cross-link bonds can be formed in any suitable manner that provides attachment of a material to an implantable frame, including formation of cross-link chemical bonds between two regions of the material . For example, cross-linking can be introduced by chemical treatment of the material, such as glycosylation. The material can be subjected to a form of energy to introduce cross-linking. For example, energy treatment suitable for use in the invention includes exposing the material to ultraviolet light, heat, or both. Dehydrothermal treatment (DHT) is one example of a physical method of crosslinking material such as an extracellular matrix material. DHT can be performed by slowly heating the material to a suitable temperature at a reduced pressure. For instance, collagen fibers can be cross-linked by heating to a temperature of about 110°C at a pressure of about 0.1µm Hg for a suitable period of time, which can be on the order of up to about 5 days. Alternatively, extracellular matrix material such as collagen can be cross-linked by irradiation at a suitable ultraviolet wavelength, such as 254 nm. For example, irradiation of collagen at 254 nm for about 15-240 minutes at a distance of about 6 inches from a UV light source. DHT and UV irradiation cross linking methods for collagen materials are further compared and discussed in Weadock, et al., "Physical crosslinking of collagen fibers: Comparison of ultraviolet irradiation and dehydrothermal treatment," J. Biomed. Mat. Res., 29:1373-1379 (1995).

In general, the material for use in the medical device and material for leaflet formation can be processed prior to cross-linking the material. For example, the material can undergo cutting and trimming, sterilizing, and associating the material with one or more desirable compositions, such as anticalcification agents and growth factors, and the like. After any preliminary processing and or storage is completed, the material can be cross-linked. Following cross-linking of the material, the material can be further processed, which can involve additional chemical and or mechanical manipulation of the material as well as processing the material into the desired medical device.

Cross-linking can be performed, for example, to mechanically stabilize the material to the device. Cross-linked material generally refers to material that is completely cross-linked in the sense that further contact with a cross-linking agent does not further change measurable mechanical properties of the material. However, total (100%) cross-linking is not always needed to achieve many desired mechanical properties. Cross-linking of the material preferably involves a chemical cross-linking agent with a plurality of functional groups that bond to the material to form a chemically cross-linked material. The chemical cross-linking is preferably performed until a cross-linking agent has permeated the material of the cross-linking region and reacted with the accessible binding sites of the material.

Once a desired level of cross-linking is reached, the mechanical properties of the material are generally determined or set. While the mechanical properties of cross-linked material are stabilized, the material may gradually change upon exposure to physiological conditions or under inappropriate storage conditions, such as dehydrating conditions for reasonable periods of time. With proper storage, the cross-linked material typically possesses suitably stable mechanical properties. Since the cross-linked material has stable mechanical properties, the mechanical and physical properties of the material can be matched, such that the properties of the respective material is within desired tolerances.

Cross-linking of natural polymers or synthetic polymers can be accomplished with lyophilization, adhesives, pressure and or/heat. Chemical cross-linking can also be used to join layers of material together. Other cross-linking agents can incorporate glutaraldehyde, albumin, formaldehyde or a combination thereof. Material can also be fixed by cross-linking. Fixation provides mechanical stabilization, for example, by preventing enzymatic degradation of the tissue and by anchoring the collagen fibrils.

Other cross-linking agents can be used to form cross-linking regions, such as epoxides, epoxyamines, diimides and other difunctional polyfunctional aldehydes. In particular, aldehyde functional groups are highly reactive with amine groups in proteins, such as collagen. Epoxyamines are molecules that generally include both an amine moiety (e.g. a primary, secondary, tertiary, or quaternary amine) and an epoxide moiety. The epoxyamine compound can be a monoepoxyamine compound and or a polyepoxyamine compound. In some embodiments, the epoxyamine compound is a polyepoxyamine compound having at least two epoxide moieties and possibly three or more epoxide moieties. In some embodiments, the polyepoxyamine compound is triglycidylamine (TGA). The use of cross-linking agents forms corresponding adducts, such as glutaraldehyde adducts and epoxyamine adducts, of the cross-linking agent with the material that have an identifiable chemical structures. Particularly preferred cross-linking agents can be selected from agents useful in cross-linking collagen, such as: diisocyanate compounds (such as hexamethylene diisocyanate), water-soluble carbodiimide (including 1-ethyl-3-(3-dimethyl aminopropyl)carbodiimide, or EDC), N-hydroxysuccinimide (NHS), a combination of EDC and NHS (also called EN-crosslinking), 1,4-butanediol diglycidyl ether (BD), acyl azide and dimethyl suberimidate (DMS). EN-crosslinking can be achieved by contacting the extracellular matrix material with a buffered solution (e.g., pH 5.5) of EDC (e.g., about 1.15 g in 100 mL) and NHS (e.g., about 0.14 g NHS in the same 100 mL) for a time period suitable to achieve crosslinking (e.g., up to about 24 hrs at 20°C). Alternatively, the material may be crosslinked by contacting a solution of Na₂B₄O₇ •10 H₂O (e.g., 0.025M at pH 9.0 or 4.5) with BD (e.g., 4g or 4 wt%) and permitting crosslinking for a desired period of time (e.g., about 144 hours). Crosslinking of collagen tissue with BD, GA and EN is described in van Wachem et al., "In Vivo Behavior of Epoxy-Crosslinked Porcine Heart Valve Cusps and Walls," J. Biomed. Mater. Res. (Appl. Biomater.) 53:18-27 (2000). Propyleneimine octaamine dendrimers may also be used as crosslinking agents with extracellular matrix material. For example, diamine(ethylene diamine), triamine (tris-2-aminoethylamine) and dendrimer-crosslinked collagens may be prepared by contacting the material with an aqueous solution of EDC and the multifunctional amine crosslinking agent and a suitablem amount of NHS. The pH of the solution can be adjusted to a desired level, such as 5.5, and crosslinking can be achieved at a suitable temperature, such as about 37°C. Preparation of dendrimer-crosslinked collagens are described in Duan et al., "Crosslinking of collagen with dendrimers," J. Biomed. Mater. Res. 75A: 510-518 (2005). The preparation of crosslinked extracellular material with DMS can be used to attach the material to a frame containing suitable nitrogen-containing reactive groups, such as amine or amidine groups. For example, collagen material may be crosslinked by contacting the material with an aqueous solution of DMS (e.g., 0.2M) at a suitable pH (e.g., about 9.0) at room temperature for a suitable period of time (e.g., 0.5, 1.0, 1.5, 2.0 and higher). Charulatha et al., "Crosslinking density and resorption of dimethyl suberimidate-treated collagen," J. Biomed. Mater. Res., 36: 478-486 (1997) described methods of crosslinking collagen with a DMS solution.

In general, the process to form completely cross-linked material is conducted for a suitable amount of time. For example, the cross-linking agent may be allowed to penetrate through the material. Also, the cross-linking process generally reaches a point of completion at which time the properties of the material are essentially stable with respect to any additional measurable changes upon further contact with the cross-linking agent. At the point of completion, it is thought that the cross-linking composition forms a stable cross-linked region. Upon completion, the cross-linking is often irreversible such that contact over significant periods of time with aqueous solutions without the cross-linking agent present does not result in reversal of the cross-linking and disassembly of the cross-linked region. Presumably, at completion, many, if not all, of the available functional groups of the material for cross-linking have reacted with a cross-linking agent. Since the formation of a fully cross-linked material is a slow process, the degree of cross-linking of the material at the cross-linking region can be selected to range from very low levels to completion of cross-linking.

Any cross-links can be made via the formation of a chemical bond such as by the reaction of a donor functional group and acceptor functional group. Acceptor functional groups include aldehydes, epoxides, anhydrides, esters, amides, carbonates, urethanes, acetals, ketals, α,β-unsaturated carbonyl compounds and the like. Donor functional groups contain oxygen, nitrogen and sulfur atoms such as alcohols, amines, sulfides, amides and the like. Materials containing nitrogen can be cross-linked with a suitable cross-linking agent containing acceptor functionality such as formaldehyde, glutaraldehyde, polyfunctional aldehydes and epoxides to form a nitrogen-carbon bond such as an aminal or alkylated amine. Glutaraldehyde and polyepoxides are particularly well suited cross-linking agents for nitrogen containing materials because they are difunctional and contain multiple aldehyde and or epoxide functionalities. Other materials containing acceptor functionality such as polyesters, polycarbonates, polyimides, polyurethanes can be cross-linked with a suitable cross-linking agent containing donor functionality such as diamines, polyamines, diols and polyols to form ester, carbonate, amide, urea, and imide bonds. Methods of forming such bonds are well known to one skilled in the art.

In a preferred embodiment, a polyurethane urea, such as the polyureaurethane material sold under the tradename Thoralon®, is cross-linked with small intestine submucosa (SIS). Cross linking of these two materials can be accomplished by reacting the ester functionality of SIS with a cross-linking agent containing an oxygen or nitrogen to form an ester or amide bond, respectively. Polyurethane ureas can be cross-linked by reaction of the urea functionality with an oxygen or nitrogen functionality to form a urea bond or urethane bond. Polyamines, polyalcohols or amino alcohols are suitable cross-linking agent to cross-link polyurethane ureas and SIS. Alternatively, an epoxy amine or epoxy alcohol could be used to cross-link Thoralon and SIS. In this case the amine or alcohol functionality of the cross-linking agent would form an ester or amide bond with the SIS material, and the epoxy functionality of the cross-linking agent would alkylate the urea functionality of the Thoralon.

The polyurethane material preferably comprises a biocompatible polyurethane material described in U.S. Pat. Application Publication No. 2002/0065552 A1 and U.S. Pat. No. 4,675,361. The biocompatible polyurethane material sold under the tradename THORALON is a polyurethane base polymer (referred to as BPS-215) blended with a siloxane containing surface modifying additive (referred to as SMA-300). The concentration of the surface modifying additive may be in the range of 0.5% to 5% by weight of the base polymer. One particularly preferred polyurethane material is sold as THORALON (THORATEC, Pleasanton, CA). The SMA-300 component (THORATEC) is a polyurethane comprising polydimethylsiloxane as a soft segment and the reaction product of diphenylmethane diisocyanate (MDI) and 1, 4-butanediol as a hard segment. A process for synthesizing SMA-300 is described, for example, in U.S. Pat. Nos. 4,861,830 and 4,675,361, which are incorporated herein by reference. The BPS-215 component (THORATEC) is a segmented polyetherurethane urea containing a soft segment and a hard segment. The soft segment is made of polytetramethylene oxide (PTMO), and the hard segment is made from the reaction of 4, 4'-diphenylmethane diisocyanate (MDI) and ethylene diamine (ED).

A variety of other biocompatible polyurethanes/polycarbamates and urea linkages (hereinafter "-C(O)N or CON-type polymers") may also be employed. These include CON type polymers that preferably include a soft segment and a hard segment. The segments can be combined as copolymers or as blends. For example, CON type polymers with soft segments such as PTMO, polyethylene oxide, polypropylene oxide, polycarbonate, polyolefin, polysiloxane (i.e. polydimethylsiloxane), and other polyether soft segments made from higher homologous series of diols may be used. Mixtures of any of the soft segments may also be used. The soft segments also may have either alcohol end groups or amine end groups. The molecular weight of the soft segments may vary from about 500 to about 5,000 g/mole. Preferably, the hard segment is formed from a diisocyanate and diamine. The diisocyanate may be represented by the formula OCN-R-NCO, where -R- may be aliphatic, aromatic, cycloaliphatic or a mixture of aliphatic and aromatic moieties. The diamine used as a component of the hard segment includes aliphatic amines, aromatic amines and amines containing both aliphatic and aromatic moieties.

Alternatively, two materials may be cross-linked using radical reactions. A radical is generated in the material to be cross-linked using a free radical generator, such as an organic peroxide of which many are known and commercially available, such as dicumyl peroxide, benzoyl peroxide, and the like. In this case the cross-linking agent is a multifunctional monomer capable of cross-linking the particular polymer when initiated by the free radical generator or irradiation. Typically, the cross-linking agent contains at least two ethylenic double bonds, which may be present, for example, in allyl, methallyl, propargyl or vinyl groups.

In general, material that corresponds to each leaflet can be separately cross-linked. To introduce a desired shape to the material, a segment of material generally is cross-linked in contact with a curved surface for at least a portion of the cross-linking period. The material is contacted with a cross-linking agent for a sufficient period of time to completely crosslink the material. The cross-linking agent can be delivered through one or more apparatuses to perform the cross-linking. The material can be, for example, immersed in the cross-linking agent during the cross-linking process and or cross-linked by spraying the cross-linking agent onto the region of material. For embodiments in which the material is immersed in a cross-linking agent with a self -polymerizing cross-linking agent, the material can be separated from the supply of cross-linking agent by a semi-permeable membrane such that higher molecular weight oligomers polymers of the cross-linking agent are blocked by the semi-permeable membrane.

### MATERIALS

A wide variety of materials can be incorporated into the medical device, including polymers, polyesters, and extracellular matrix proteins, such as collagen, gelatin, elastin, glycoproteins, silk collagen/elastic and combinations thereof. Material preferably comprises a cross-linkable polymer. Material is preferably flexible, such as a valve leaflet or portion of biological tissue valve. Material is preferably capable of forming cross link bonds.

The material is also preferably biocompatible, or able to be made biocompatible, and be capable of being cross-linked. Examples of suitable materials include naturally derived materials, and synthetic materials. Examples of suitable natural materials include collagen and extracellular matrix (ECM) material, such as submucosa. Small intestine submucosa (SIS) is particularly well-suited as a material, such as to form valve leaflets. Examples of suitable synthetic materials include polymeric materials, such as polypropylene, polyurethane, expanded polytetrafluoroethylene (ePTFE), polyurethane (PU), polyethylene terphthalate (PET), silicone, latex, polyethylene, polypropylene, polycarbonate, nylon; polytetrafluoroethylene, polyimide, polyester, and mixture thereof, or other Suitable materials.

In one embodiment, the material can comprise a substantially biocompatible material, such as polyester fabrics, polytetrafluoroethylene (PTFE), expanded PTFE, and other synthetic materials known to those of skill in the art. In one embodiment, the material can comprise a naturally occurring biomaterial, such as collagen. In one embodiment, the material comprises a collagen material known as extracellular matrix (ECM). In one embodiment, a suitable ECM is small intestinal submucosa (SIS). Other examples of ECMs are pericardium, stomach submucosa, liver basement membrane, urinary bladder submucosa, tissue mucosa, and dura mater.

In one embodiment, the material can comprise a remodelable material. The terms "remodelable" or "bioremodelable" refer to the ability of a material to allow or induce host tissue growth, proliferation or regeneration following implantation of the material in vivo. Remodelling can occur in various microenvironments within a body, including without limitation soft tissue, a sphincter muscle region, body wall, tendon, ligament, bone and cardiovascular tissues. Upon implantation of a remodelable material, cellular infiltration and neovascularization are typically observed over a period of about five days to about six months or longer, as the remodelable material acts as a matrix for the ingrowth of adjacent tissue with site-specific structural and functional properties. The remodeling phenomenon which occurs in mammals following implantation of submucosal tissue includes rapid neovascularization and early mononuclear cell accumulation. Mesenchymal and epithelial cell proliferation and differentiation are typically observed by one week after in vivo implantation and extensive deposition of new extracellular matrix occurs almost immediately. In some embodiments, fluid contacting autologous cells on an implanted remodelable material can affect the growth of autologous tissue on the implanted remodelable material.

One example of a suitable remodelable material useful as a material is extracellular matrix (ECM) material derived from submocosal tissue. Submucosal tissue can be obtained from various tissue sources, harvested from animals raised for meat production, including, for example, pigs, cattle and sheep or other mammals. More particularly, the submucosa is isolated from warm-blooded tissues including the alimentary, respiratory, intestinal, urinary or genital tracts of warm-blooded vertebrates.

One preferred category of ECM material is submucosal tissue. Submucosal ECM material can be obtained from any suitable source, including without limitation, intestinal submucosa, stomach submucosa, urinary bladder submucosa, and uterine submucosa. Intestinal submucosal tissue is one preferred starting material, and more particularly intestinal submucosa delaminated from both the tunica muscularis and at least the tunica mucosa of warm-blooded vertebrate intestine. More preferably, the ECM material is Tela submucosa, which is a layer of collagen-containing connective tissue occurring under the mucosa in most parts of the alimentary, respiratory, urinary and genital tracts of animals. Examples of suitable ECM materials include renal capsule matrix (RCM), urinary bladder matrix (UBM) and most preferably small intestine submucosa (SIS). Most preferably, the ECM material is obtained from processed intestinal collagen layer derived from the tunic submucosa of porcine small intestine.

"Tela submucosa" refers to a layer of collagen-containing connective tissue occurring under the mucosa in most parts of the alimentary, respiratory, urinary, integumentary, and genital tracts of animals. Tela submucosa, as with many animal tissues, is generally aseptic in its natural state, provided the human or animal does not have an infection or disease. This is particularly the case since the tela submucosa is an internal layer within the alimentary, respiratory, urinary and genital tracts of animals. Accordingly, it is generally not exposed to bacteria and other cellular debris such as the epithelium of the intestinal tract. Preferably, the tela submucosa tissue ECM materials, which are collagen-based and thus predominantly collagen, are derived from the alimentary tract of mammals and most preferably from the intestinal tract of pigs. A most preferred source of whole small intestine is harvested from mature adult pigs weighing greater than about 450 pounds. Intestines harvested from healthy, nondiseased animals will contain blood vessels and blood supply within the intestinal tract, as well as various microbes such as *E*. *coli* contained within the lumen of the intestines. Therefore, disinfecting the whole intestine prior to delamination of the tela submucosa substantially removes these contaminants and provides a preferred implantable tela submucosa tissue which is substantially free of blood and blood components as well as any other microbial organisms, pyrogens or other pathogens that may be present. In effect, this procedure is believed to substantially preserve the inherent aseptic state of the tela submucosa, although it should be understood that it is not intended that the present invention be limited by any theory.

Additional information as to submucosa materials useful as ECM materials herein can be found in U.S. Patent Nos. 4,902,508; 5,554,389; 5,993,844; 6,206,931; 6,099,567; and 6,375,989, as well as published U.S. Patent Applications US2004/0180042A1 and US2004/0137042A1. For example, the mucosa can also be derived from vertebrate liver tissue as described in WIPO Publication, WO 98/25637, based on PCT application PCT/US97/22727; from gastric mucosa as described in WIPO Publication, WO 98/26291, based on PCT application PCT/US97/22729; from stomach mucosa as described in WIPO Publication, WO 98/25636, based on PCT application PCT/US97/23010; or from urinary bladder mucosa as described in U.S. Pat. No. 5,554,389.

A material can comprise an ECM material isolated from biological tissue by a variety of methods. In general, an ECM material can be obtained from a segment of intestine that is first subjected to abrasion using a longitudinal wiping motion to remove both the outer layers (particularly the tunica serosa and the tunica muscularis) and the inner layers (the luminal portions of the tunica mucosa). Typically the SIS is rinsed with saline and optionally stored in a hydrated or dehydrated state until use as described below. The resulting submucosa tissue typically has a thickness of about 100-200 micrometers, and may consist primarily (greater than 98%) of acellular, eosinophilic staining (H&E stain) ECM material.

Preferably, the source tissue for the ECM material is a tela submucosa that is disinfected prior to delamination by the preparation disclosed in US Patent Application US2004/0180042A1 by Cook et al., published September 16, 2004. Most preferably, the tunica submucosa of porcine small intestine is processed in this manner to obtain the ECM material. This method is believed to substantially preserve the aseptic state of the tela submucosa layer, particularly if the delamination process occurs under sterile conditions. Specifically, disinfecting the tela submucosa source, followed by removal of a purified matrix including the tela submucosa, e.g. by delaminating the tela submucosa from the tunica muscularis and the tunica mucosa, minimizes the exposure of the tela submucosa to bacteria and other contaminants. In turn, this enables minimizing exposure of the isolated tela submucosa matrix to disinfectants or sterilants if desired, thus substantially preserving the inherent biochemistry of the tela submucosa and many of the tela submucosa's beneficial effects.

Preferably, the ECM material is substantially free of any antibiotics, antiviral agents or any antimicrobial type agents which may affect the inherent biochemistry of the matrix and its efficacy upon implantation. An alternative to the preferred method of ECM material isolation comprises rinsing the delaminated biological tissue in saline and soaking it in an antimicrobial agent, for example as disclosed in U.S. Pat. No. 4,956,178. While such techniques can optionally be practiced to isolate ECM material from submucosa, preferred processes avoid the use of antimicrobial agents and the like which may not only affect the biochemistry of the collagen matrix but also can be unnecessarily introduced into the tissues of the patient.

Other disclosures of methods for the isolation of ECM materials include the preparation of intestinal submucosa described in U.S. Pat. No. 4,902,508. Urinary bladder submucosa and its preparation is described in U.S. Pat. No. 5,554,389. Stomach submucosa has also been obtained and characterized using similar tissue processing techniques, for example as described in U.S. patent application Ser. No. 60/032,683 titled STOMACH SUBMUCOSA DERIVED TISSUE GRAFT, filed on Dec. 10, 1996.

In some embodiments, submucosal tissues for use in accordance with this invention include intestinal submucosa, stomach submucosa, urinary bladder submucosa, and uterine submucosa. Another specific example of a suitable remodelable material is intestinal submucosal tissue, and more particularly intestinal submucosa delaminated from both the tunica muscularis and at least the tunica mucosa of warm-blooded vertebrate intestine.

SIS can be made, for example, in the fashion described in U.S. Patent No. 4,902,508 to Badylak et al., U.S. Patent No. 5,733,337 to Carr, and WIPO Patent No. WO 9822158, published May 28, 1998, issued to Cook Biotech Inc. et al. and listing Patel et al. as inventors. The preparation and use of SIS is also described in U.S. Pat. Nos. 5,281,422 and 5,275,826. Urinary bladder submucosa and its preparation is described in U.S. Pat. No. 5,554,389. The use of submucosal tissue in sheet form and fluidized forms for inducing the formation of endogenous tissues is described and claimed in U.S. Pat. Nos. 5,281,422 and 5,275,826.

A variety of remodelable materials are known in the art, including naturally derived or synthetic collagenous materials that are capable of providing remodelable surfaces on implantable medical devices. Examples of material materials useful in certain embodiments of the present invention also include: the regenerative compositions comprising epithelial basement membranes as described in U.S. Patent No. 6,579,538 to Spievack, the remodelable implantable valves described in U.S. Patent No. 6,126,686 to Badylak et al., the enzymatically digested submucosal gel matrix composition of U.S. Patent No. 6,444,229 to Voytik-Harbin et al., materials comprising the carboxy-terminated polyester ionomers described in U.S. Patent No. 5,668,288 to Storey et al., the biodegradable surgical implant of U.S. Patent No. 6,171,338 to Talja et al., collagen-based matrix structure described in U.S. Patent No. 6,334,872 to Termin et al., the autologous tissue venous valve described in U.S. Patent No. 6,562,068 to Drasler et al., and combinations thereof.

Although certain embodiments of the present invention provide a material comprising remodelable material, the invention is not limited to material materials that are remodelable. Any material known in the art that provides a desirable property that is responsive to fluid contacting the surface when implanted *in vivo* can be used to provide a material.

Any suitable implantable material or portion of an implantable material, including those described above, can serve as the material. The examples recited above provide illustrative examples of some suitable materials that can be selected to provide a material. Other materials known in the art can also be selected to provide a material for some embodiments of the present invention.

### FRAMES

Any suitable support frame can be used as the support frame in the medical device. The specific support frame chosen will depend on several considerations, including the size and configuration of the vessel and the size and nature of the medical device.

A support frame that provides a stenting function, i.e., exerts a radially outward force on the interior of the body vessel in which the medical device is implanted, can be used if desired. By including a support frame that provides a stenting function, the medical device can provide a stenting functionality at a point of treatment in a body vessel. The stent art provides numerous examples of support frames acceptable for use in the medical device, and any suitable stent can be used as the support frame. If a stent is used, the specific stent chosen will depend on several factors, including the vessel in which the medical device is being implanted, the axial length of the treatment site, the number of valves desired in the device, the inner diameter of the body vessel, the delivery method for placing the support structure, and others. Those skilled in the art can determine an appropriate stent based on these and other factors.

The support frame can have any suitable size. The exact configuration and size chosen will depend on several factors, including the desired delivery technique, the nature of the body vessel in which the medical device will be implanted, and the size of the vessel. The support frame can be sized so that the second, expanded configuration is slightly larger in diameter that the inner diameter of the vessel in which the medical device will be implanted. This sizing can facilitate anchoring of the medical device within the body vessel and maintenance of the medical device at a point of treatment following implantation.

Suitable support frames can also have a variety of configurations, including braided strands, helically wound strands, ring members, consecutively attached ring members, tube members, and frames cut from solid tubes. Also, suitable frames can have a variety of sizes. The exact configuration and size chosen will depend on several factors, including the desired delivery technique, the nature of the vessel in which the device will be implanted, and the size of the vessel. A frame structure and configuration can be chosen to facilitate maintenance of the device in the vessel following implantation.

The support frame can be made from one or more suitable materials. Examples of suitable materials include, without limitation: stainless steel (such as 316 stainless steel), nickel titanium (NiTi) alloys (such as Nitinol) and other shape memory and/or superelastic materials, MP35N, gold, silver, a cobalt-chromium alloy, tantalum, platinum or platinum iridium, or other biocompatible metals and/or alloys such as carbon or carbon fiber, cellulose acetate, cellulose nitrate, silicone, cross-linked polyvinyl alcohol (PVA) hydrogel, cross-linked PVA hydrogel foam, polyurethane, polyamide, styrene isobutylene-styrene block copolymer (Kraton), polyethylene teraphthalate, polyurethane, polyamide, polyester, polyorthoester, polyanhidride, polyether sulfone, polycarbonate, polypropylene, high molecular weight polyethylene, polytetrafluoroethylene, or other biocompatible polymeric material, or mixture of copolymers thereof, or stainless steel, polymers, and any suitable composite material.

In one embodiment, the frame is self-expanding. Upon compression, self-expanding frames can expand toward their precompression geometry. In some embodiments, a self-expanding frame can be compressed into a low-profile delivery conformation and then constrained within a delivery system for delivery to a point of treatment in the lumen of a body vessel. At the point of treatment, the self-expanding frame can be released and allowed to subsequently expand to another configuration. In certain embodiments, the frame is formed partially or completely of alloys such as nitinol (NiTi) which have superelastic (SE) characteristics. However, while some embodiments provide frames made from shape memory materials, other embodiments comprise other materials such as stainless steel, MP35N and other suitable materials. Some embodiments provide frames that are not self-expanding, or that do not comprise superelastic materials.

The support frame can be formed in any suitable shape, including a ring, a stent, a tube, or a zig-zag configuration. In one embodiment, the support frame can be self-expanding or balloon-expandable.

The support frame can be formed from a variety of medical grade polymers having properties that permit the frame to function as a supporting structure for the remodelable material. In some embodiments, the support frame comprises a bioabsorbable or remodelable material.

The support frame can comprise a bioabsorbable material that can be degraded and absorbed by the body over time to advantageously eliminate a frame structure from the vessel before, during or after the remodeling process. A number of bioabsorbable homopolymers, copolymers, or blends of bioabsorbable polymers are known in the medical arts. These include, but are not necessarily limited to, polyesters, poly(amino acids), copoly(ether-esters), polyalkylenes oxalates, polyamides, poly(iminocarbonates), polyorthoesters, polyoxaesters, polyamidoesters, polyoxaesters containing amido groups, poly(anhydrides), polyphosphazenes, poly-alpha-hydroxy acids, trimethlyene carbonate, poly-beta-hydroxy acids, polyorganophosphazines, polyanhydrides, polyesteramides, polyethylene oxide, polyester-ethers, polyphosphoester, polyphosphoester urethane, cyanoacrylates, poly(trimethylene carbonate), poly(iminocarbonate), polyalkylene oxalates, polyvinylpyrolidone, polyvinyl alcohol, poly-N-(2-hydroxypropyl)-methacrylamide, polyglycols, aliphatic polyesters, poly(orthoesters), poly(ester-amides), polyanhydrides, modified polysaccharides and modified proteins.

Some specific examples of bioabsorbable materials include polymers and co-polymers comprising a polylactic acid, a polyglycolic acid, a polycaprolactone or derivatives thereof. Suitable bioabsorbable materials for a frame include: poly(epsilon-caprolactone), poly(dimethyl glycolic acid), poly(hydroxy butyrate), poly(p-dioxanone), polydioxanone, PEO/PLA, PLA, poly(lactide-co-glycolide), poly(hydroxybutyrate-co-valerate), poly(glycolic acid-co-trimethylene carbonate), poly(epsilon-caprolactone-co-p-dioxanone), poly-L-glutamic acid or poly-L-lysine, polylactic acid, polylactide, polyglycolic acid, polyglycolide, poly(D,L-lactic acid), L-polylactic acid, poly(glycolic acid), polyhydroxyvalerate, cellulose, chitin, dextran, fibrin, casein, fibrinogen, starch, collagen, hyaluronic acid, hydroxyethyl starch, and gelatin. A frame may also comprise one or more naturally derived bioabsorbable polymers, including modified polysaccharides such as cellulose, chitin, and dextran or modified proteins such as fibrin and casein.

The frame can include structural features, such as barbs, that maintain the frame in position following implantation in a body vessel. The art provides a wide variety of structural features that are acceptable for use in the medical device, and any suitable structural feature can be used. Furthermore, barbs can also comprise separate members attached to the frame by suitable attachment means, such as welding and bonding. For instance, barbs can be formed by V-shaped cuts transversing the thickness of a flat metal frame, which are bent outward to form the barb. In some embodiments, the number, arrangement, and configuration of the integral barbs can vary according to design preference and the clinical use of the device. The barbs can have any suitable shape, including points or "fish hook"-like configurations. The barbs may or may not penetrate the vein wall, depending on their design and other factors, including the thickness and type of covering used.

Also provided are embodiments wherein the frame comprises a means for orienting the frame within a body lumen. For example, the frame can comprise a marker, such as a radiopaque portion of the frame that would be seen by remote imaging methods including X-ray, ultrasound, Magnetic Resonance Imaging and the like, or by detecting a signal from or corresponding to the marker. In other embodiments, the delivery device can comprise a frame with indicia relating to the orientation of the frame within the body vessel. In other embodiments, indicia can be located, for example, on a portion of a delivery catheter that can be correlated to the location of the frame within a body vessel.

A frame or delivery device may comprise one or more radiopaque materials to facilitate tracking and positioning of the medical device, which may be added in any fabrication method or absorbed into or sprayed onto the surface of part or all of the medical device. The degree of radiopacity contrast can be altered by implant content. Radiopacity may be imparted by covalently binding iodine to the polymer monomeric building blocks of the elements of the implant. Common radiopaque materials include barium sulfate, bismuth subcarbonate, and zirconium dioxide. Other radiopaque elements include: cadmium, tungsten, gold, tantalum, bismuth, platinum, iridium, and rhodium. In one preferred embodiment, iodine may be employed for its radiopacity and antimicrobial properties. Radiopacity is typically determined by fluoroscope or x-ray film. Various other ways to incorporate radiopaque material in a medical device are provided in copending application 10/787,307, filed February 26, 2004 by Case et al., entitled "Prosthesis Adapted for Placement Under External Imaging" . Imagable markers, including radiopaque material, can be incorporated in any portion of a medical device. For example, radiopaque markers can be used to identify a long axis or a short axis of a medical device within a body vessel. For instance, radiopaque material may be attached to a frame or woven into portions of the valve member material.

### DELIVERY

Preferably, the medical device is implanted in a compressed configuration, and expanded at a point of treatment within a body vessel. The overall configuration, cross-sectional area, and length of the frame in the tubular configuration (compressed or expanded) will depend on several factors, including the size and configuration of device, the size and configuration of the vessel in which the device will be implanted, the extent of contact between the device and the walls of the vessel, and the amount of retrograde flow through the vessel that is desired.

Although the medical device is shown with two valve members, other embodiments provide medical devices comprising 1, 3, 4, 5, 6, 7, 8 or more valve members. The valve members can be arranged in any suitable configuration with respect to one another and the frame. In one preferred embodiment, a medical device can comprise a frame and three valve members that are leaflets comprising free edges. In another preferred embodiment, a medical device can comprise one leaflet having a free edge that can sealably engage the interior of a vessel wall. Other suitable configurations of valve members are provided by further embodiments, including differently shaped valve members, and different points of attachment by valve members to the frame.

In one embodiment, the valve members are substantially oriented parallel to the longitudinal axis of a medical device. The orientation of a valve member in a medical device is discussed in U.S. patent application Ser. No. 10/787,307, filed February 26, 2004 (by Case et al.), entitled "Prosthesis Adapted for Placement Under External Imaging".

In devices including multiple openings that permit a controlled amount of fluid flow in the second, opposite direction to flow through the vessel in which the device is implanted, the total open area of all openings can be optimized as described above, but it is not necessary that the individual openings have equivalent total open areas.

### METHODS OF MANUFACTURING

In some embodiments, the medical devices can be configured for delivery to a body vessel. For example, a medical device can be compressed to a delivery configuration within a retaining sheath that is part of a delivery system, such as a catheter-based system. Upon delivery, the delivery configuration can be expanded, for example, by removing a self-expanding frame, or portion thereof, from the sheath or by inflating a balloon from inside the medical device. The delivery configuration can be maintained prior to deployment of the medical device by any suitable means, including a sheath, a suture, a tube or other restraining material around all or part of the compressed medical device, or other methods. A method of making an implantable medical device can comprise providing a frame and covering the frame with a cross-linkable material. The method can further comprise cross-linking at least one region of the cross-linkable material, wherein the cross-linking comprises forming a cross-linked region joining a first region of the cross-linkable material with a second region of the cross-linkable material.

In some embodiments, a bioabsorbable suture or sheath can be used to maintain a medical device in a compressed configuration both prior to and after deployment. As the bioabsorbable sheath or suture is degraded by the body after deployment, the medical device can expand within the body vessel. In some embodiments, a portion of the medical device can be restrained with a bioabsorbable material and another portion allowed to expand immediately upon implantation. For example, a self-expanding frame can be partially restrained by a bioabsorbable material upon deployment and later expand as the bioabsorbable material is absorbed.

The invention also provides methods of making medical devices for implantation in a body vessel. In one embodiment, the method comprises the step of attaching a first valve member to a frame. The valve member can be responsive to the flow of fluid through the frame, and adapted to permit fluid flow through said vessel in a first direction or substantially prevent fluid flow through said vessel in a second, opposite direction. The frame can have a longitudinal axis, a first radial compressibility along a first radial direction that is less than a second radial compressibility along a second radial direction.

### METHODS OF TREATMENT

The invention also provides methods of treating a patient. In one embodiment the method comprises a step of delivering a medical device as described herein to a point of treatment in a body vessel, and deploying the medical device at the point of treatment. Additionally, the medical device can comprise a frame and a cross-linkable material having a cross-linked region where the cross-linked region maintains the material in connection to the frame.

The delivering step can comprise delivery by surgical or by percutaneous delivery techniques known to those skilled in the art.

Still other embodiments provide methods of treating a subject, which can be animal or human, comprising the step of providing one or more frames as described herein. Other methods further comprise the step of providing one or more frames attached to one or more valve members, as described herein. In some embodiments, methods of treating may also provide the step of delivering a medical device to a point of treatment in a body vessel, or deploying a medical device at the point of treatment, wherein the medical devices are as described herein.

Methods for treating certain conditions are also provided, such as venous valve insufficiency, varicose veins, esophageal reflux, restinosis or atherosclerosis. In some embodiments, the invention relates to methods of treating venous valve related conditions.

A "venous valve related condition" is any condition presenting symptoms that can be diagnostically associated with improper function of one or more venous valves. In mammalian veins, natural valves are positioned along the length of the vessel in the form of leaflets disposed annularly along the inside wall of the vein which open to permit blood flow toward the heart and close to prevent back flow. These natural venous valves act as open to permit the flow of fluid in the desired direction, and close upon a change in pressure, such as a transition from systole to diastole. When blood flows through the vein, the pressure forces the valve leaflets apart as they flex in the direction of blood flow and move towards the inside wall of the vessel, creating an opening therebetween for blood flow. The leaflets, however, do not normally bend in the opposite direction and therefore return to a closed position to restrict or prevent blood flow in the opposite, i.e. retrograde, direction after the pressure is relieved. The leaflets, when functioning properly, extend radially inwardly toward one another such that the tips contact each other to block backflow of blood. Two examples of venous valve related conditions are chronic venous insufficiency and varicose veins.

In the condition of venous valve insufficiency, the valve leaflets do not function properly. For example, the vein can be too large in relation to the leaflets so that the leaflets cannot come into adequate contact to prevent backflow (primary venous valve insufficiency), or as a result of clotting within the vein that thickens the leaflets (secondary venous valve insufficiency). Incompetent venous valves can result in symptoms such as swelling and varicose veins, causing great discomfort and pain to the patient. If left untreated, venous valve insufficiency can result in excessive retrograde venous blood flow through incompetent venous valves, which can cause venous stasis ulcers of the skin and subcutaneous tissue. Venous valve insufficiency can occur, for example, in the superficial venous system, such as the saphenous veins in the leg, or in the deep venous system, such as the femoral and popliteal veins extending along the back of the knee to the groin.

The varicose vein condition consists of dilatation and tortuosity of the superficial veins of the lower limb and resulting cosmetic impairment, pain and ulceration. Primary varicose veins are the result of primary incompetence of the venous valves of the superficial venous system. Secondary varicose veins occur as the result of deep venous hypertension which has damaged the valves of the perforating veins, as well as the deep venous valves. The initial defect in primary varicose veins often involves localized incompetence of a venous valve thus allowing reflux of blood from the deep venous system to the superficial venous system. This incompetence is traditionally thought to arise at the saphenofemoral junction but may also start at the perforators. Thus, gross saphenofemoral valvular dysfunction may be present in even mild varicose veins with competent distal veins. Even in the presence of incompetent perforation, occlusion of the saphenofemoral junction usually normalizes venous pressure.

The initial defect in secondary varicose veins is often incompetence of a venous valve secondary to hypertension in the deep venous system. Since this increased pressure is manifested in the deep and perforating veins, correction of one site of incompetence could clearly be insufficient as other sites of incompetence will be prone to develop. However, repair of the deep vein valves would correct the deep venous hypertension and could potentially correct the secondary valve failure. Apart from the initial defect, the pathophysiology is similar to that of varicose veins.

Methods for delivering a medical device as described herein to any suitable body vessel are also provided, such as a vein, artery, billiary duct, ureteral vessel, body passage or portion of the alimentary canal.

## Claims

1. A medical device (10) for implantation in a body vessel, comprising:
a frame (20); and
a cross-linkable material (30) having a cross-linked region (40) and a non cross-linked region, wherein the cross-linked region includes cross-linkable bonds between first and second regions (32, 34) of the cross-linkable material at a plurality of attachment points (50), said first and second regions attaching the cross-linkable material to itself around the frame, the cross-linked region maintaining the cross-linkable material in connection to the frame and wherein the cross-linkable material is movable relative to the frame.

2. A medical device as claimed in claim 1, wherein the attachment points (50) are discrete longitudinally along the frame (20).

3. A medical device as claimed in claim 1 or 2, wherein a plurality of non-crosslinked regions is interposed with the plurality of discrete points of attachment.

4. A medical device as claimed in any preceding claim, wherein the cross-linked region of each point of attachment is formable by treatment with a chemical cross-linking agent.

5. A medical device as claimed in claim 4, wherein the chemical cross-linking agent is selected from the group consisting of glutaraldehyde, albumin, formaldehyde, epoxides, epoxyamines, diimides, diisocyanate compounds, water soluble carbodiimides, N-hydroxysuccinimide, 1,4-butanediol diglycidyl ether, acyl azides, dimethyl suberimidate, and combinations thereof.

6. A medical device as claimed in any preceding claim, wherein the cross-linkable material comprises an extracellular matrix material.

7. A medical device as claimed in any preceding claim, wherein the cross-linkable material comprises submucosal tissue.

8. A medical device as claimed in any preceding claim, wherein the cross-linkable material comprises small intestine submucosa.

9. A medical device as claimed in any one of claims 1 to 4, wherein the cross-linkable material comprises a polymer.

10. A medical device claimed in any one of claims 1 to 4, wherein the cross-linkable material comprises a cross-linkable tissue.

11. A medical device claimed in any one of claims 1 to 4, wherein the cross-linkable material comprises a material that allows or induces host tissue growth, proliferation or regeneration following implantation of the material in vivo.

12. A medical device as claimed in claim 1, wherein the cross-linked region comprises cross-link bonds between a sugar and a protein.

13. A medical device claimed in any preceding claim, wherein the frame comprises:
a material selected from the group consisting of, a nickel titanium alloy, a cobalt chromium alloy, a polymeric material, and a bioabsorbable material; or
a material selected from the group consisting of nickel, titanium, and stainless steel.

14. A medical device as claimed in claim 1, wherein the frame comprises a cross-linkable polymer or small intestine submucosa.

## Patentansprüche

1. Medizinische Vorrichtung (10) zur Implantation in ein Körpergefäß, die Folgendes umfasst:
einen Rahmen (20); und
ein vernetzbares Material (30) mit einer vernetzten Region (40) und einer nicht vernetzten Region, worin die vernetzte Region vernetzbare Verbindungen zwischen ersten und zweiten Regionen (32, 34) des vernetzbaren Materials an einer Vielzahl von Befestigungspunkten (50) aufweist, wobei die ersten und zweiten Regionen das vernetzbare Material um den Rahmen an sich selbst befestigen, wobei die vernetzte Region das vernetzbare Material mit dem Rahmen in Verbindung hält und worin das vernetzbare Material relativ zum Rahmen bewegt werden kann.

2. Medizinische Vorrichtung nach Anspruch 1, worin die Befestigungspunkte (50) in Längsrichtung auf dem Rahmen (20) diskret angeordnet sind.

3. Medizinische Vorrichtung nach Anspruch 1 oder 2, worin eine Vielzahl von unvernetzten Regionen zwischen die Vielzahl von diskreten Befestigungspunkten eingeschoben ist.

4. Medizinische Vorrichtung nach einem der vorhergehenden Ansprüche, worin die unvernetzte Region jedes Befestigungspunkts durch Behandlung mit einem chemischen Vernetzungsmittel geformt werden kann.

5. Medizinische Vorrichtung nach Anspruch 4, worin das chemische Vernetzungsmittel aus der aus Glutaraldehyd, Albumin, Formaldehyd, Epoxide, Epoxyamine, Diimide, Diisocyanat-Verbindungen, wasserlösliche Carbodiimide, N-Hydroxysuccinimid, 1,4-Butandioldiglycidylether, Acylazide, Dimethylsuberimidat und Kombinationen davon bestehenden Gruppe ausgewählt ist.

6. Medizinische Vorrichtung nach einem der vorhergehenden Ansprüche, worin das vernetzbare Material ein extrazelluläres Matrixmaterial umfasst.

7. Medizinische Vorrichtung nach einem der vorhergehenden Ansprüche, worin das vernetzbare Material Submukosagewebe umfasst.

8. Medizinische Vorrichtung nach einem der vorhergehenden Ansprüche, worin das vernetzbare Material Dünndarm-Submukosa umfasst.

9. Medizinische Vorrichtung nach einem der Ansprüche 1 bis 4, worin das vernetzbare Material ein Polymer umfasst.

10. Medizinische Vorrichtung nach einem der Ansprüche 1 bis 4, worin das vernetzbare Material ein vernetzbares Gewebe umfasst.

11. Medizinische Vorrichtung nach einem der Ansprüche 1 bis 4, worin das vernetzbare Material ein Material umfasst, das Einwachsen von Wirtsgewebe, Proliferation oder Regeneration nach Implantation des Materials in vivo gestattet.

12. Medizinische Vorrichtung nach Anspruch 1, worin die vernetzte Region Vernetzungen zwischen einem Zucker und einem Protein umfasst.

13. Medizinische Vorrichtung nach einem der vorhergehenden Ansprüche, worin der Rahmen Folgendes umfasst:
ein aus der aus einer Nickel-Titan-Legierung, einer Kobalt-Chrom-Legierung, einem Polymermaterial und einem biologisch resorbierbaren Material bestehenden Gruppe ausgewähltes Material; oder
ein aus der aus Nickel, Titan und Edelstahl bestehenden Gruppe ausgewähltes Material umfasst.

14. Medizinische Vorrichtung nach Anspruch 1, worin der Rahmen ein vernetzbares Polymer oder Dünndarmsubmukosa umfasst.

## Revendications

1. Dispositif médical (10) à implanter dans un vaisseau corporel, comprenant:
un cadre (20); et
un matériau réticulable (30) qui présente une région réticulée (40) et une région non réticulée, dans lequel la région réticulée comprend des liaisons réticulables entre des première et deuxième régions (32, 34) du matériau réticulable en une pluralité de points de fixation (50), lesdites première et deuxième régions attachant le matériau réticulable à lui-même autour du cadre, la région réticulée maintenant le matériau réticulable en connexion avec le cadre, et dans lequel le matériau réticulable est mobile par rapport au cadre.

2. Dispositif médical selon la revendication 1, dans lequel les points de fixation (50) sont disposés de façon discrète longitudinalement le long du cadre (20).

3. Dispositif médical selon la revendication 1 ou 2, dans lequel une pluralité de régions non réticulées sont intercalées avec la pluralité de points de fixation discrets.

4. Dispositif médical selon l'une quelconque des revendications précédentes, dans lequel les régions réticulées de chaque point de fixation peuvent être formées par un traitement avec un agent de réticulation chimique.

5. Dispositif médical selon la revendication 4, dans lequel l'agent de réticulation chimique est sélectionné dans le groupe comprenant le glutaraldéhyde, l'albumine, le formaldéhyde, les époxydes, les époxyamines, les diimides, les composés de diisocyanate, les carbodiimides solubles dans l'eau, le N-hydroxysuccinimide, le 1,4-butane-diol diglydicyle éther, les azides acyles, le subérimidate de diméthyle ainsi que des combinaisons de ceux-ci.

6. Dispositif médical selon l'une quelconque des revendications précédentes, dans lequel le matériau réticulable comprend un matériau de matrice extra-cellulaire.

7. Dispositif médical selon l'une quelconque des revendications précédentes, dans lequel le matériau réticulable comprend un tissu sub-mucosal.

8. Dispositif médical selon l'une quelconque des revendications précédentes, dans lequel le matériau réticulable comprend une petite sous-muqueuse intestinale.

9. Dispositif médical selon l'une quelconque des revendications 1 à 4, dans lequel le matériau réticulable comprend un polymère.

10. Dispositif médical selon l'une quelconque des revendications 1 à 4, dans lequel le matériau réticulable comprend un tissu réticulable.

11. Dispositif médical selon l'une quelconque des revendications 1 à 4, dans lequel le matériau réticulable comprend un matériau qui permet ou induit une croissance, une prolifération ou une régénération du tissu hôte après l'implantation du matériau *in vivo.*

12. Dispositif médical selon la revendication 1, dans lequel la région réticulée comprend des liaisons de réticulation entre un sucre et une protéine.

13. Dispositif médical selon l'une quelconque des revendications précédentes, dans lequel le cadre comprend:
un matériau qui est sélectionné dans le groupe comprenant un alliage de nickel et de titane, un alliage de cobalt et de chrome, un matériau polymère et un matériau biorésorbable; ou
un matériau qui est sélectionné dans le groupe comprenant le nickel, le titane et l'acier inoxydable.

14. Dispositif médical selon la revendication 1, dans lequel le cadre comprend un polymère réticulable ou une petite sous-muqueuse intestinale.
